# EUROPEAN PATENT APPLICATION

(11) **EP 4 736 938 A1**
(43) Date of publication of application: **06.05.2026**
(21) Application number: 24875888.0
(22) Date of filing: 23.01.2024
(51) Int. Cl.: A61N 5/02

(54) **MULTI-PROBE INTELLIGENT MICROWAVE PHYSIOTHERAPY INSTRUMENT**

(30) Priority: 10.10.2023 CN 202311304848
(71) Applicant: Henan Lixinghe Medical Technology Co., Ltd., Kaifeng, Henan 475300 (CN)
(72) Inventor: ZHANG, Yanming, Kaifeng, Henan 475300 (CN)
(74) Representative: Feder Walter Ebert
(86) International application number: PCT/CN2024/073539
(87) International publication number: WO 2025/077058

(57) **Abstract**

The present invention relates to the technical field of microwave physiotherapy instruments. Disclosed is a multi-probe intelligent microwave physiotherapy instrument. Pressure-bearing air bags are fixedly connected between the tops of physiotherapy probes and the top of an inner cavity of a protective shell. The inner side wall of a circulation plate is uniformly provided with second circulation holes, and the outer side wall of the circulation plate is sleeved with an expansion air bag, the expansion air bag communicating with the second circulation holes by means of an air conveying pipe. An air blowing disc is fixedly mounted on the bottom of the protective shell, the pressure-bearing air bags being connected to the air blowing disc by means of air guide pipes. Pressure electromagnetic valves are fixedly mounted on the air guide pipes, an electric control valve is fixedly mounted on the air conveying pipe, and air feeding one-way valves are fixedly mounted on the pressure-bearing air bags. Thus, physiotherapy assemblies can be in contact with the surface of the skin instead of the physiotherapy probes and finally can reach the state of being attached to the surface of the skin. Therefore, treatment can be uniformly applied to the physiotherapy site so as to timely reduce the heat brought to the skin surface, thereby achieving automatic cooling and preventing the skin of patients from burning.

## Description

### TECHNICAL FIELD

The present disclosure relates to the technical field of microwave physiotherapy instruments, in particular to an multi-probe intelligent microwave physiotherapy instrument.

### BACKGROUND

Microwave refers to the electromagnetic wave with the frequency of 300 MHz-300 GHz, which is the abbreviation of a limited frequency band in radio waves, i.e., the electromagnetic wave with a wavelength ranging from 1 mm to 1 m. The frequency of microwave is higher than that of ordinary radio waves, and thus the microwave is usually called "UHF electromagnetic wave". The microwave, as an electromagnetic wave, also has wave-particle duality. The basic properties of the microwave usually show three characteristics: penetration, reflection and absorption.

For example, a microwave physiotherapy acupoint acupuncture device and a use method thereof are provided in CN115400016A. The microwave physiotherapy acupoint acupuncture device includes a main console, and a physiotherapy couch. An end part of the physiotherapy couch is provided with a mounting frame and a control box, the mounting frame is provided with a patient parameter display screen, a microwave source is arranged inside the control box, and a control panel is arranged outside the control box. A side part of the physiotherapy couch is provided with a mounting box, a microwave magnetron is arranged inside the mounting box, and a mechanical arm is arranged at a top of the mounting box. An end part of the mechanical arm is provided with a microwave probe, and the microwave magnetron is connected to the microwave probe at the end part of the mechanical arm through a line. According to the microwave physiotherapy acupoint acupuncture device and the use method thereof, compared with the existing microwave acupuncture equipment, an integrated microwave physiotherapy acupoint acupuncture device structure is employed, when a patient lies on a physiotherapy couch, ten acupoints of the patient can be subjected to microwave physiotherapy at the same time, and a lying posture of the patient can be adjusted through an electric physiotherapy couch, thus further improving the effect of microwave physiotherapy.

The ten acupoints of the patient can be subjected to microwave physiotherapy at the same time through the above technical solution, thus the effect of microwave physiotherapy is further improved. However, when the probe of the physiotherapy instrument is used for physiotherapy on human skin, the probe is attached to the surface of human skin, and the probe of the physiotherapy instrument is at a constant temperature, but the human skin is soft and uneven, when the probe is used for physiotherapy on uneven sites, the physiotherapy parts cannot be uniformly treated. Moreover, when the probe of the physical therapy instrument is used for physical therapy on the side of the human body and other curved sites, the skin surface is not evenly heated, which will affect the effect of microwave physiotherapy, and when the probe is attached to the skin surface at the same temperature for a long time, a situation that the skin feels burning may be caused, which cannot be relieved in time through the adjustment of the machine.

Therefore, it is necessary to solve the above problem by an multi-probe intelligent microwave physiotherapy instrument.

### SUMMARY

An objective of the present disclosure is to provide a support device for an multi-probe intelligent microwave physiotherapy instrument to solve the problems in the background above.

To achieve the objective above, the present disclosure provides the following technical solutions: An multi-probe intelligent microwave physiotherapy instrument includes a main console, a physiotherapy table, and an operation console. The physiotherapy table is located at one side of the main console, the operation console is located at one side of the physiotherapy table, and multiple first operating arms are rotatably arranged at one side of the operation console. The multiple first operating arms are arranged in a linear array, the other end of the first operating arm is hinged with a second operating arm, and the other end of the second operating arm is fixedly assembled with an adjusting component. A physiotherapy probe is slidably assembled in the adjusting component, multiple groups of physiotherapy components with the same structure are inserted into the bottom of the adjusting component, and the physiotherapy component corresponds to the physiotherapy probe. A controller is fixedly assembled in the main console.

A pressure-bearing gasbag is fixedly connected between the top of the physiotherapy probe and the top of an inner cavity of a protective shell, a flow-through plate is integrally formed on an outer edge of the bottom of the physiotherapy probe, second flow-through holes are uniformly formed in an inner side wall of the flow-through plate, and an outer side wall of the flow-through plate is sleeved with an expansion gasbag. The expansion gasbag communicates with the second flow-through hole through a gas conveying pipe. The bottom of the protective shell is fixedly assembled with a gas-blowing disc, the pressure-bearing gasbag is connected to the gas-blowing disc through a gas guiding pipe, and a pressure solenoid valve is fixedly installed on the gas guiding pipe. An electric control valve is fixedly assembled on the gas conveying pipe, and a gas inlet check valve is fixedly assembled on the pressure-bearing gasbag.

A support spring is connected between the top of the physiotherapy component and the physiotherapy probe, an amount of compression of the supporting spring is used to detect a distance between the physiotherapy probe and human skin, thus determining human skin-conformable condition of the physiotherapy component.

Preferably, the adjusting component includes a protective shell, a driving motor is fixed to the top of the physiotherapy probe, an output end of the driving motor is fixedly assembled with a threaded rod, and a limiting plate is fixed to the other end of the threaded rod. The threaded rod is threaded to the protective shell, and a limiting rod fixed to the top of the physiotherapy probe is inserted into the top of the protective shell.

Preferably, the physiotherapy probe includes a probe housing, and a microwave component is arranged in the probe housing. A first connector is threaded to one side of the probe housing, a gasket is connected to one side of the first connector, the driving motor is fixed to the top of the probe housing, the limiting rod is fixed to the other side of the top of the probe housing, and a second connector is inserted in the middle of the probe housing. The second connector is fixed to the protective shell, and is hinged with an end of the second operating arm through a ball joint.

Preferably, the gasket is located between the first connector and the microwave component, a connecting hole is formed in the middle of the top of the probe housing, and the second connector is inserted into the connecting hole.

Preferably, a flow-through slot communicating with the expansion gasbag is formed in an outer side wall of the protective shell, and a rotating hole in fit with the limiting rod is formed in the top of the protective shell.

Preferably, multiple indicator lamps are fixedly arranged on one side of the operation console, the multiple indicator lamps are arranged in a linear array, and the indicator lamps correspond to the first operating arms in positions.

Preferably, the physiotherapy component includes multiple contacts, and a cavity is left in each contact. A first flow-through hole is formed in one side of the contact, a limiting ring is fixedly connected to an outer surface of the contact, and one side, away from the first flow-through hole, of the contact is provided with a rotating slot. A limiting block is fixedly connected to one side, close to the rotating slot, of an inner arm of the contact, and a rotating ball is rotatably arranged in the contact. The contacts are arranged in a circumferential array, and a temperature sensor is fixedly arranged on a middle group of contacts.

Preferably, multiple sliding holes are formed in the bottom of the protective shell, a position of the sliding hole is in fit with the contact, and the contact slides in the sliding hole.

Preferably, the gas-blowing disc is attached to the bottom of the protective shell, vent holes are uniformly formed in the gas-blowing disc, and the vent holes communicate with the gas guiding pipe.

Preferably, a total of four groups of support springs is provided, and a single group of support springs is fixed to the top of a group of physiotherapy components at the outermost side of each of the front, back, left, and right.

The technical effect and advantages of the present disclosure are as follows:
1. The physiotherapy component provided by the present disclosure is in contact with the skin surface instead of the physiotherapy probe, and as the contacts are slidably connected to the protective shell, the contacts may be attached to the uneven skin surface at different heights, making each contact in contact with the skin, such that the treatment uniformity of the skin surface at this site can be better. Moreover, the heat is generated at a physiotherapy site during physiotherapy, which can promote the blood circulation of the human body. When the physiotherapy probe is moved, the rotating ball rotates accordingly, which not only facilitates the movement of the physiotherapy probe, but also plays the role of massage during movement, thus promoting the blood circulation of the human body.
2. The distance between the physiotherapy probe and the human skin can be detected by the amount of compression of the support spring, such that a thermal field radiation distance of the physiotherapy probe can be intuitively detected, and the height position of the physiotherapy probe can be conveniently adjusted in real time. During the use of the four groups of support springs, as long as the amount of compression occurs, it is indicated that the contacts are completely attached to the human skin at this time. When the amount of compression of the support springs reaches the maximum, it is indicated that the radiation distance of the thermal field of the physiotherapy probe to the human skin is the nearest, and the physiotherapy heat is the largest. However, as long as the amount of compression of one group of supporting springs is 0, it is indicated that the contact is about to be separated from the human skin at this time, and a conformable state of the physiotherapy component to the human skin may also be detected based on the compression of the supporting springs.
3. The temperature sensor can detect the temperature radiated by the thermal field of the physiotherapy probe to the human skin at all time, so the physiotherapy on the human body can be well controlled. The height position of the physiotherapy probe can be adjusted by the driving motor, and in the adjustment process, different cooling operations and heating operations are implemented based on the change of the amount of compression of the supporting springs to ensure that the human skin does not feel burning at a high temperature and avoid the problem of poor physiotherapy at a low temperature.

### BRIEF DESCRIPTION OF THE DRAWINGS

FIG. 1 is a schematic diagram of an overall structure according to the present disclosure;
FIG. 2 is a sectional diagram of a physiotherapy probe and a flow-through plate according to the present disclosure;
FIG. 3 is a first sectional diagram of a physiotherapy probe according to the present disclosure;
FIG. 4 is a second sectional diagram of a physiotherapy probe according to the present disclosure;
FIG. 5 is a schematic diagram of an assembly structure of a physiotherapy probe and an adjusting component according to the present disclosure;
FIG. 6 is a schematic diagram of an assembly structure of an adjusting component and a physiotherapy component according to the present disclosure;
FIG. 7 is a first sectional diagram of a physiotherapy probe, an adjusting component, and a physiotherapy component according to the present disclosure;
FIG. 8 is a second sectional diagram of a physiotherapy probe, an adjusting component, and a physiotherapy component according to the present disclosure;
FIG. 9 is a second sectional diagram of a physiotherapy probe, an adjusting component, and a physiotherapy component according to the present disclosure.
FIG. 10 is a sectional diagram of a contact according to the present disclosure.

In the drawings: 1-main console; 2-physiotherapy table; 3-operation console; 301-first operating arm; 302-second operating arm; 303-indicator lamp; 4-physiotherapy probe; 401-probe housing; 402-microwave component; 403-first connector; 404-gasket; 405-second connector; 406-connecting hole; 5-physiotherapy component; 501-contact; 502-first flow-through hole; 503-limiting ring; 504-rotating slot; 505-limiting block; 506-rotating ball; 507-temperature sensor; 508-support spring; 6-adjusting component; 601-flow-through plate; 602-second flow-through hole; 603-protective shell; 604-flow-through slot; 605-gas-blowing disc; 606-rotating hole; 607-driving motor; 6071-threaed rod; 6072-limiting plate; 608-limiting rod; 609-pressure-bearing gasbag; 610-expansion gasbag.

### DETAILED DESCRIPTION OF THE EMBODIMENTS

The following clearly and completely describes the technical solutions in the embodiments of the present disclosure with reference to the accompanying drawings in the embodiments of the present disclosure. Apparently, the accompanying drawings in the following description show merely some embodiments of the present disclosure, and those of ordinary skill in the art may still derive other drawings from these accompanying drawings without creative efforts.

### Embodiment 1

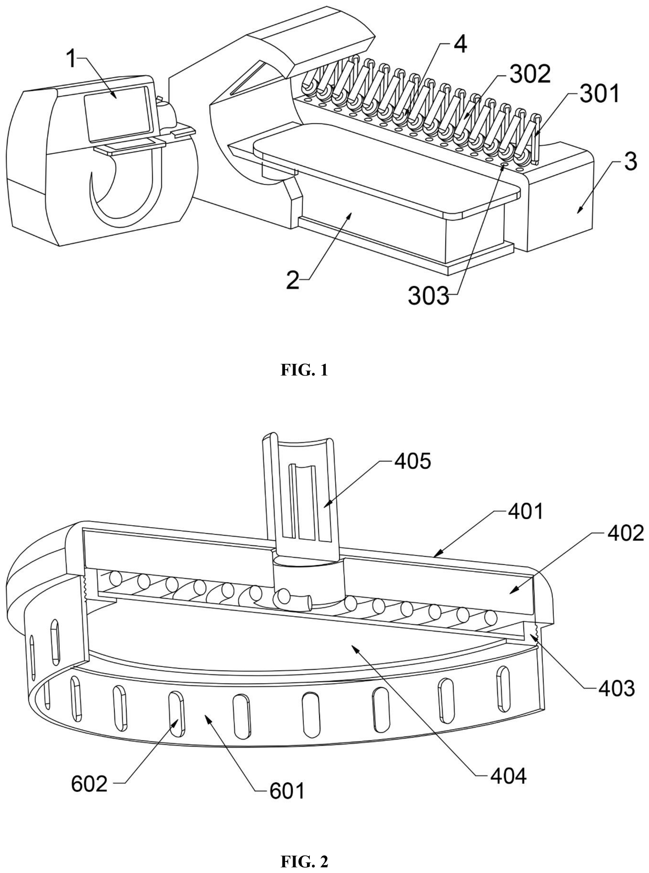

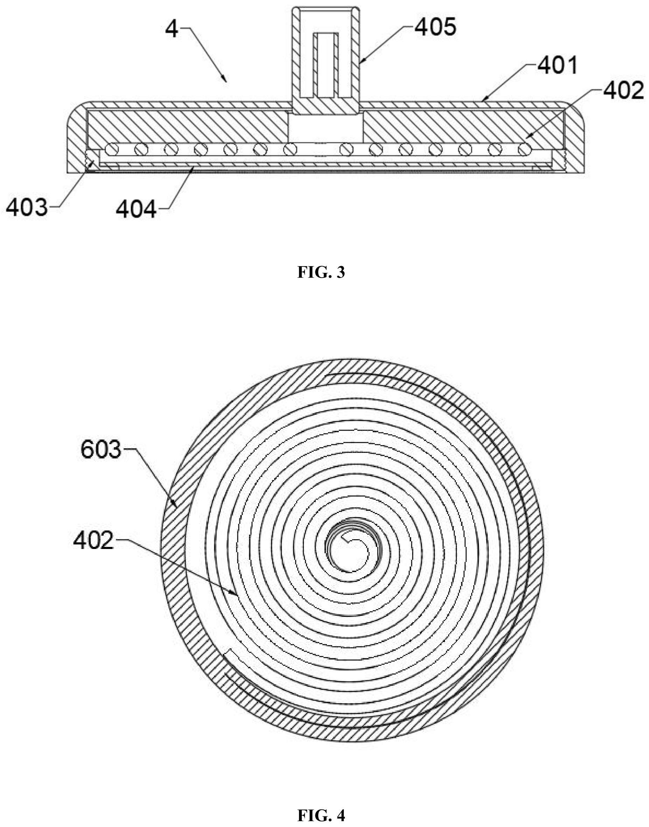

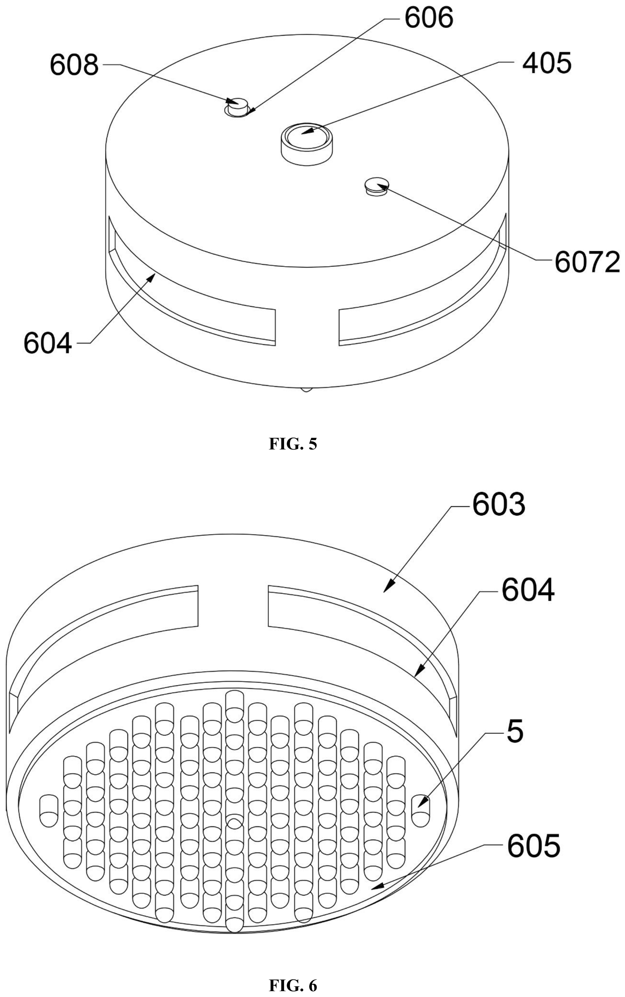

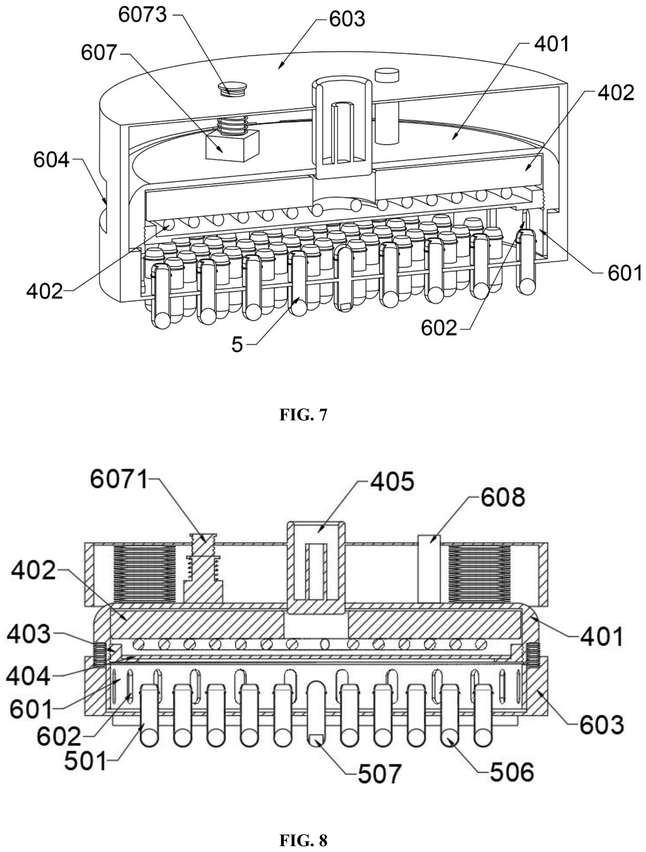

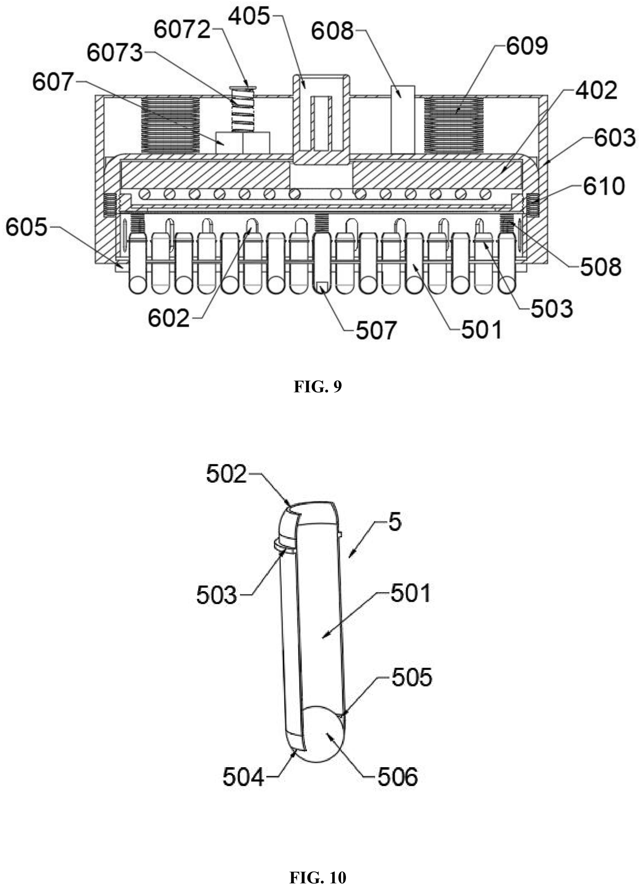

The present disclosure provides an multi-probe intelligent microwave physiotherapy instrument shown in FIG. 1 to FIG. 10, including a main console 1, a physiotherapy table 2, and an operation console 3. The physiotherapy table 2 is located at one side of the main console 1, the operation console 3 is located at one side of the physiotherapy table 2, and multiple first operating arms 301 are rotatably arranged at one side of the operation console 3. The multiple first operating arms 301 are arranged in a linear array, the other end of the first operating arm 301 is hinged with a second operating arm 302, and the other end of the second operating arm 302 is fixedly assembled with an adjusting component 6. A physiotherapy probe 4 is slidably assembled in the adjusting component 6, multiple groups of physiotherapy components 5 with the same structure are inserted into the bottom of the adjusting component 6, and the physiotherapy component 5 corresponds to the physiotherapy probe 4. A controller is fixedly assembled in the main console 1.

During use, when a patient lies on the physiotherapy table 2, the physiotherapy table 2 and the operation console 3 are operated numerically through the main console 1, and an operator can stand on one side of the operation console 3 to operate and adjust the first operating arm 301 and the physiotherapy probe 4.

The adjusting component 6 includes a protective shell 603, a driving motor 607 is fixed to the top of the physiotherapy probe 4, an output end of the driving motor 607 is fixedly assembled with a threaded rod 6071, and a limiting plate 6072 is fixed to the other end of the threaded rod 6071. The threaded rod 6071 is threaded to the protective shell 603, and a limiting rod 608 fixed to the top of the physiotherapy probe 4 is inserted into the top of the protective shell 603.

A support spring 508 is connected between the top of the physiotherapy component 5 and the physiotherapy probe 4, an amount of compression of the supporting spring 508 is used to detect a distance between the physiotherapy probe 4 and human skin, thus determining human skin-conformable condition of the physiotherapy component 5.

The physiotherapy probe 4 includes a probe housing 401, and a microwave component 402 is arranged in the probe housing 401. A first connector 403 is threaded to one side of the probe housing 401, a gasket 404 is connected to one side of the first connector 403, the driving motor 607 is fixed to the top of the probe housing 401, the limiting rod 608 is fixed to the other side of the top of the probe housing 401, and a second connector 405 is inserted in the middle of the probe housing 401. The second connector 405 is fixed to the protective shell 603, and is hinged with an end of the second operating arm 302 through a ball joint.

The gasket 404 is located between the first connector 403 and the microwave component 402, a connecting hole 406 is formed in the middle of the top of the probe housing 401, and the second connector 405 is inserted into the connecting hole 406.

A flow-through slot 604 communicating with the expansion gasbag 610 is formed in an outer side wall of the protective shell 603, and a rotating hole 606 in fit with the limiting rod 608 is formed in the top of the protective shell 603.

Multiple indicator lamps 303 are fixedly arranged on one side of the operation console 3, the multiple indicator lamps 303 are arranged in a linear array, and the indicator lamps 303 correspond to the first operating arms 301 in positions. During use, the indicator lamps 303 correspond to the physiotherapy probes 4 in positions and numbers. When the physiotherapy probe 4 is used, the corresponding indicator light 303 will be lit, such that the operator can clearly know the use of the physiotherapy probe 4, and the operation efficiency of the operator is improved.

The physiotherapy component 5 includes multiple contacts 501, and a cavity is left in each contact 501. A first flow-through hole 502 is formed in one side of the contact 501, a limiting ring 503 is fixedly connected to an outer surface of the contact 501, and one side, away from the first flow-through hole 502, of the contact 501 is provided with a rotating slot 504. A limiting block 505 is fixedly connected to one side, close to the rotating slot 504, of an inner arm of the contact 501, and a rotating ball 506 is rotatably arranged in the contact 501. The contacts 501 are arranged in a circumferential array, and a temperature sensor 507 is fixedly arranged on a middle group of contacts 501.

During use, as the physiotherapy component 5 is connected to the physiotherapy probe 4, the physiotherapy component 5 is in contact with the skin surface instead of the physiotherapy probe 4, and as the contacts 501 are slidably connected to the protective shell 603, the contacts 501 may be attached to the uneven skin surface at different heights, making each contact 501 in contact with the skin, such that the treatment uniformity of the skin surface at this site can be better. Moreover, the heat is generated at a physiotherapy site during physiotherapy, which can promote the blood circulation of the human body. The cavity is left in the contact 501, the first flow-through hole 502 is formed in one side of the contact 501, and thus the heat generated by the microwave component 402 can be transmitted to the skin through the cavity. When the physiotherapy probe 4 is moved, the rotating ball 506 rotates accordingly, which not only facilitates the movement of the physiotherapy probe 4, but also plays the role of massage during movement, thus promoting the blood circulation of the human body.

The temperature sensor 507 can detect the temperature on the skin surface of human at all time to prevent the temperature from being too high or too low.

Multiple sliding holes are formed in the bottom of the protective shell 603, and the position of the sliding hole is in fit with the contact 501. The contact 501 slides in the sliding hole, such that the contact 501 can slide at the bottom of the protective shell 603 to make the contact 501 have higher skin-conformal degree.

During use, when the patient lies on the physiotherapy table 2, the physiotherapy table 2 and the operation console 3 are operated numerically through the main console 1, and the operator can stand on one side of the operation console 3 to operate and adjust the first operating arm 301 and the second operating arm 302, thus promoting the contact to be attached to the physiotherapy site. Then, the physiotherapy probe 4 is controlled to start working, which makes the microwave component 402 generate heat, and the heat can be transmitted to the physiotherapy site through the contact 501, such that different sites can feel the heat for physiotherapy, and the effect of uniform physiotherapy is achieved. The temperature sensor 507 can monitor the temperature of the physiotherapy site in real time, and when the temperature is too high, the controller will control the driving motor 607 to work to drive the threaded rod 6071 to rotate, and the protective shell 603 is driven to move in a height direction through the threaded connection between the threaded rod 6071 and the protective shell 603. Due to the fact that the limiting rod 608 has a limiting effect on the protective shell 603, and the second connector 405 is fixedly connected to the protective shell 603, when the driving motor 607 rotates forward, the physiotherapy probe 4 moves upwards, and when the driving motor 607 rotates reversely, the physiotherapy probe 4 moves downwards, such that the height position of the physiotherapy probe 4 can be adjusted according to the temperature change during physiotherapy, the thermal field position of the physiotherapy probe 4 can be adjusted to achieve the adjustment of the temperature on human skin and prevent scalding. When the thermal field position of the physiotherapy probe 4 changes, a gap between the protective shell 603 and the bottom of the physiotherapy probe 4 becomes larger, such that the outside air can flow in, and the heat in the space is diluted through the increase of the air volume, thus achieving the corresponding cooling effect.

### Embodiment 2

In the working process of Embodiment 1, because the thermal field position is changed only by adjusting the height of the physiotherapy probe 4, which is only the adjustment of the radiation distance of the thermal field. However, when the temperature in the protective shell 603 is too high, the temperature cannot be quickly reduced by simple adjustment. If the physiotherapy probe 4 is taken away by the second operating arm 302, the physiotherapy component 5 cannot be in contact with the human skin at this time, and thus the physiotherapy probe 4 cannot achieve the physiotherapy effect. Meanwhile, when the height position of the physiotherapy probe 4 is adjusted, the volume of gas introduced into the protective shell 603 is small, and the air circulation speed is slow, so the operability of cooling based on air flow is small, and the cooling effect is poor. Based on this, the present disclosure proposes the following improvement solution.

A pressure-bearing gasbag 609 is fixedly connected between the top of the physiotherapy probe 4 and the top of an inner cavity of a protective shell 603, a flow-through plate 601 is integrally formed on an outer edge of the bottom of the physiotherapy probe 4, second flow-through holes 602 are uniformly formed in an inner side wall of the flow-through plate 601, and an outer side wall of the flow-through plate 601 is sleeved with an expansion gasbag 610. The expansion gasbag 610 communicates with the second flow-through hole 602 through a gas conveying pipe. The bottom of the protective shell 603 is fixedly assembled with a gas-blowing disc 605, the pressure-bearing gasbag 609 is connected to the gas-blowing disc 605 through a gas guiding pipe, and a pressure solenoid valve is fixedly installed on the gas guiding pipe. An electric control valve is fixedly assembled on the gas conveying pipe, and a gas inlet check valve is fixedly assembled on the pressure-bearing gasbag 609.

The gas-blowing disc 605 is attached to the bottom of the protective shell 603, vent holes are uniformly formed in the gas-blowing disc 605, and the vent holes communicate with the gas guiding pipe.

A total of four groups of support springs 508 is provided, and a single group of support springs 508 is fixed to the top of a group of physiotherapy components 5 at the outermost side of each of the front, back, left and right. During actual use, after the contact 501 is in contact with the human skin, an upward pressure is applied, and the 501 retracts into the protective shell 603 at this time. Meanwhile, the four groups of support springs 508 are compressed, such that a thermal field radiation distance of the physiotherapy probe 4 can be intuitively detected, and the height position of the physiotherapy probe can be conveniently adjusted in real time. During the use of the four groups of support springs 508, as long as the amount of compression occurs, it is indicated that the contacts 501 are completely attached to the human skin at this time. When the amount of compression of the support springs 508 reaches the maximum, it is indicated that the radiation distance of the thermal field of the physiotherapy probe 4 to the human skin is the nearest, and the physiotherapy heat is the largest. However, as long as the amount of compression of one group of supporting springs 508 is 0, it is indicated that the contact 501 is about to be separated from the human skin at this time, and a conformable state of the physiotherapy component 5 to the human skin may also be detected based on the compression of the supporting springs 508.

During actual use, the temperature sensor 507 can detect the temperature radiated by the thermal field of the physiotherapy probe 4 to the human skin at all time, so the physiotherapy on the human body can be well controlled. When the temperature sensor 507 detects that the temperature exceeds a standard threshold, a cooling operation is required, that is, the controller controls the driving motor 607 to rotate reversely, which can drive the physiotherapy probe 4 to move upward to achieve an adjustment operation of increasing the distance of the thermal field, and thus the temperature adjustment can be fundamentally achieved.

In this process, the upward movement of the physiotherapy probe 4 will cause the pressure-bearing gasbag 609 to be compressed, and the amount of compression of the support spring 508 will gradually decrease, and meanwhile, the expansion gasbag 610 is stretched and expanded, such that the height adjustment of the thermal field of the physiotherapy probe 4 can be achieved by the upward movement of the physiotherapy probe 4, and the initial cooling operation can be achieved by the air flowing into the protective shell 603. When the amount of compression of the support spring 508 is reduced to a first threshold, the temperature of the temperature sensor 507 has not decreased to the standard threshold, and in this case, the controller still controls the physiotherapy probe 4 to move upwards, and the controller controls the electric control valve to be opened, such that the negative pressure in the expansion gasbag 610 makes the gas in the flow-through plate 601 drive heat to be introduced into the expansion gasbag 610 through the second flow-through hole 602 and the gas conveying pipe, which can reduce the temperature in the flow-through plate 601, and achieve the secondary cooling operation.

When the amount of compression of the support spring 508 is reduced to a second threshold, the temperature of the temperature sensor has not decreased to a standard threshold value, and the controller still controls the device to perform the secondary cooling operation above. In this case, the pressure in the pressure-bearing gasbag 609 reaches a threshold of the pressure solenoid valve, the compressed gas in the pressure-bearing gasbag 609 is introduced into the gas-blowing disc 605 through the gas guiding pipe to blow onto the skin, such that the human skin can be cooled, and the burning sensation of human skin at a high temperature can be reduced.

When the amount of compression of the support spring 508 is 0, the physiotherapy probe 4 is about to leave the human skin surface. If the temperature detected by the temperature sensor 507 has not reached the standard threshold, the controller controls an alarm to alert a worker to carry out maintenance.

During actual use, when the temperature sensor 507 detects that the temperature is lower than the standard threshold, it is necessary to carry out the heating operation, that is, the controller controls the driving motor 607 to rotate forward, which can drive the physiotherapy probe 4 to move downwards to achieve the adjustment operation of reducing the distance of the thermal field, and thus the heating operation can be fundamentally achieved.

When the physiotherapy probe 4 moves downwards, the pressure-bearing gasbag 609 can achieve gas supply based on a gas check valve, which is convenient for the device to make more gas blown to human skin from the gas-blowing disc 605 during cooling operation, thus improving the cooling effect and the protection of human skin. In this process, the expansion gasbag 610 will be compressed, and the gas in the expansion gasbag 610 will be led out from the second flow-through hole 602 through the gas conveying pipe, which can accelerate the gas circulation speed in the flow-through plate 601, thus accelerating the flow of heat when the air flows, improving the circulation efficiency of heat on the human skin, and improving the physiotherapy effect.

Finally, it should be noted that the above embodiments are only preferred embodiments of the present disclosure and are not intended to limit the present disclosure. Although the present disclosure has been described in detail with reference to above embodiments, those skilled in the art still can modify the technical solutions recorded in the above embodiments, or replace some technical features by equivalents. Any modification, equivalent replacement, improvement, etc. made within the spirit and principle of the present disclosure should be included in the scope of protection of the present disclosure.

## Claims

1. An multi-probe intelligent microwave physiotherapy instrument, comprising a main console (1), a physiotherapy table (2), and an operation console (3), wherein the physiotherapy table (2) is located at one side of the main console (1), the operation console (3) is located at one side of the physiotherapy table (2), and a plurality of first operating arms (301) are rotatably arranged at one side of the operation console (3); the plurality of first operating arms (301) are arranged in a linear array, the other end of the first operating arm (301) is hinged with a second operating arm (302), and the other end of the second operating arm (302) is fixedly assembled with an adjusting component (6); a physiotherapy probe (4) is slidably assembled in the adjusting component (6), multiple groups of physiotherapy components (5) with the same structure are inserted into the bottom of the adjusting component (6), the physiotherapy component (5) corresponds to the physiotherapy probe (4), and a controller is fixedly assembled in the main console (1);
a pressure-bearing gasbag (609) is fixedly connected between the top of the physiotherapy probe (4) and the top of an inner cavity of a protective shell (603), a flow-through plate (601) is integrally formed on an outer edge of the bottom of the physiotherapy probe (4), second flow-through holes (602) are uniformly formed in an inner side wall of the flow-through plate (601), and an outer side wall of the flow-through plate (601) is sleeved with an expansion gasbag (610); the expansion gasbag (610) communicates with the second flow-through hole (602) through a gas conveying pipe; the bottom of the protective shell (603) is fixedly assembled with a gas-blowing disc (605), the pressure-bearing gasbag (609) is connected to the gas-blowing disc (605) through a gas guiding pipe, and a pressure solenoid valve is fixedly installed on the gas guiding pipe; and an electric control valve is fixedly assembled on the gas conveying pipe, and a gas inlet check valve is fixedly assembled on the pressure-bearing gasbag (609);
a support spring (508) is connected between the top of the physiotherapy component (5) and the physiotherapy probe (4), an amount of compression of the supporting spring (508) is used to detect a distance between the physiotherapy probe (4) and human skin, thus determining human skin-conformable condition of the physiotherapy component (5).

2. The multi-probe intelligent microwave physiotherapy instrument according to claim 1, wherein the adjusting component (6) comprises a protective shell (603), a driving motor (607) is fixed to the top of the physiotherapy probe (4), an output end of the driving motor (607) is fixedly assembled with a threaded rod (6071), and a limiting plate (6072) is fixed to the other end of the threaded rod (6071); the threaded rod (6071) is threaded to the protective shell (603), and a limiting rod (608) fixed to the top of the physiotherapy probe (4) is inserted into the top of the protective shell (603).

3. The multi-probe intelligent microwave physiotherapy instrument according to claim 2, wherein the physiotherapy probe (4) comprises a probe housing (401), and a microwave component (402) is arranged in the probe housing (401); a first connector (403) is threaded to one side of the probe housing (401), a gasket (404) is connected to one side of the first connector (403), the driving motor (607) is fixed to the top of the probe housing (401), the limiting rod (608) is fixed to the other side of the top of the probe housing (401), and a second connector (405) is inserted in the middle of the probe housing (401); and the second connector (405) is fixed to the protective shell (603), and is hinged with an end of the second operating arm (302) through a ball joint.

4. The multi-probe intelligent microwave physiotherapy instrument according to claim 3, wherein the gasket (404) is located between the first connector (403) and the microwave component (402), a connecting hole (406) is formed in the middle of the top of the probe housing (401), and the second connector (405) is inserted into the connecting hole (406).

5. The multi-probe intelligent microwave physiotherapy instrument according to claim 3, wherein a flow-through slot (604) communicating with the expansion gasbag (610) is formed in an outer side wall of the protective shell (603), and a rotating hole (606) in fit with the limiting rod (608) is formed in the top of the protective shell (603).

6. The multi-probe intelligent microwave physiotherapy instrument according to claim 1, wherein a plurality of indicator lamps (303) are fixedly arranged on one side of the operation console (3), the plurality of indicator lamps (303) are arranged in a linear array, and the indicator lamps (303) correspond to the first operating arms (301) in positions.

7. The multi-probe intelligent microwave physiotherapy instrument according to claim 1, wherein the physiotherapy component (5) comprises a plurality of contacts (501), and a cavity is left in each contact (501); a first flow-through hole (502) is formed in one side of the contact (501), a limiting ring (503) is fixedly connected to an outer surface of the contact (501), and one side, away from the first flow-through hole (502), of the contact (501) is provided with a rotating slot (504); a limiting block (505) is fixedly connected to one side, close to the rotating slot (504), of an inner arm of the contact (501), and a rotating ball (506) is rotatably arranged in the contact (501); and the contacts (501) are arranged in a circumferential array, and a temperature sensor (507) is fixedly arranged on a middle group of contacts (501).

8. The multi-probe intelligent microwave physiotherapy instrument according to claim 7, wherein a plurality of sliding holes are formed in the bottom of the protective shell (603), a position of the sliding hole is in fit with the contact (501), and the contact (501) slides in the sliding hole.

9. The multi-probe intelligent microwave physiotherapy instrument according to claim 1, wherein the gas-blowing disc (605) is attached to the bottom of the protective shell (603), vent holes are uniformly formed in the gas-blowing disc (605), and the vent holes communicate with the gas guiding pipe.

10. The multi-probe intelligent microwave physiotherapy instrument according to claim 1, wherein a total of four groups of support springs (508) is provided, and a single group of support springs (508) is fixed to the top of a group of physiotherapy components (5) at the outermost side of each of the front, back, left and right.
